Europäisches Patentamt

European Patent Office

Office européen des brevets

(11) Publication number: **0 315 248 B1**

# EUROPEAN PATENT SPECIFICATION

(45) Date of publication of patent specification: **15.04.92**  (51) Int. Cl.5: **C07D 301/12**, C07D 303/04

(21) Application number: **88202370.8**

(22) Date of filing: **25.10.88**

(54) **Preparation of epoxy compounds with cis- and trans-configuration, by starting from mixtures of the corresponding olefin stereoisomers.**

(30) Priority: **02.11.87 IT 2248587**

(43) Date of publication of application:
**10.05.89 Bulletin  89/19**

(45) Publication of the grant of the patent:
**15.04.92 Bulletin  92/16**

(84) Designated Contracting States:
**AT BE CH DE ES FR GB GR LI LU NL SE**

(56) References cited:
**EP-A- 0 100 119**

**TETRAHEDRON LETTERS, vol. 27, no. 6, 1986, pages 707-710, Pergamon Press Ltd, GB; D. PRAT et al.: "Epoxidations with 30% hydrogen peroxide catalyzed by tungstic acid in buffered media"**

(73) Proprietor: **ENIRICERCHE S.p.A.**
**Corso Venezia 16**
**I-20121 Milan(IT)**

(72) Inventor: **Clerici, Mario Gabriele**
**Via Europa 34**
**I-20097 San Donato Milanese Milan(IT)**
Inventor: **Bellussi, Giuseppe**
**Via Alberto Scoto 44**
**I-29100 Piacenza(IT)**

(74) Representative: **Roggero, Sergio et al**
**Ing. Barzanò & Zanardo Milano S.p.A. Via Borgonuovo 10**
**I-20121 Milano(IT)**

## Description

This invention relates to a process for separately preparing "cis" and "trans epoxy derivatives of a mixture of "cis" and "trans" isomers of unsubstituted alkenes by epoxidizing such a mixture with hydrogen peroxide.

EP-A-100 119 teaches that epoxidation of olefine compounds by epoxidation with hydrogen peroxide can be efficiently catalyzed by titanium-containing synthetic zeolites.

When the starting compound is a mixture of "cis" and "trans" isomers of unsubstituted alkenes, the final epoxidation product is a mixture of like isomers.

This inventions has as its objective the separate preparation of the "cis" and the "trans" epoxy derivatives of a mixture of "cis" and "trans" unsubstituted alkenes.

To solve this problem, the present invention provides a process for separately preparing the "cis" and the "trans epoxy derivatives of a mixture of "cis" and "trans" isomers of unsubstituted alkenes by epoxidizing such a mixture with hydrogen peroxide, or with a hydrogen-peroxide-evolving compound, in the presence of an epoxidation catalyst, characterized in that the epoxidation catalyst is a synthetic zeolite having the formula:

$$x.TiO_2.(1-x)SiO_2$$

wherein $x$ is from 0,0001 to 0,04 , and in that the process is carried out stepwise, in the first step with an amount of hydrogen peroxide near to the molar amount of the "cis" isomer of the starting isomer mixture, whereby a predominantly "cis" epoxy derivative is obtained which, in the second step, is separated from the unreacted predominantly "trans" unsubstituted alkenes, these latter being subsequently independently subjected to epoxidation with hydrogen peroxide in a nearly equimolecular amount relative to said unreacted predominantly "trans" unsubstituted alkenes.

Preferably, the amount of hydrogen peroxide in the first reaction step is from 0,85 mol to 1,5 mol per mol of the starting "cis"isomeric unsubstituted alkenes.

Preferably, the amount of hydrogen peroxide in the second reaction step is from 0,85 mol to 1,5 mol per mol of the "trans" isomeric unsubstituted alkenes recovered from the first reaction step.

The epoxidation temperature is from -20°C to 100°C, the preferred range being from 0°C to 70°C.

Hydrogen peroxide can be used as an aqueous solution, the concentration being preferably of from 10% to 70% $H_2O_2$ on a weight/volume basis.

As a general rule, the epoxidation reaction is carried out in a polar solvent medium selected from the alcohols, the glycols, the ketones, the ethers, the esters and water.

Exemplary starting unsubstituted alkenes are butene-2, pentene-2 and octene-2.

The catalyst based on titanium-containing zeolite can be prepared, for example, according to the teachings of IT-A-22608 A/82 and IT-A-20487 A/85.

In the case of volatile starting unsubstituted alkenes, the epoxidation reactions can be carried out under a pressure which may areach a value as high as 5066,16 kPa (50 atmospheres).

The separation of the epoxy compounds obtained from the epoxidation reaction from the unreacted unsubstituted alkenes can be carried out, advantageously, by distillation: this step, in the case of high-boiling products and/or reactants, can be carried out under reduced pressures.

The following examples are illustrative of the invention.

### Example 1

A titanium-silicalite zeolite is prepared as follows: 4,160 g of tetra-ethyl-silicate is prepared in a pyrex-glass beaker, and to it first 137 g of tetra-ethyl-titanate, then 7,310 g of an aqueous solution at 25% by weight of tetrapropyl-ammonium hydroxide are added with stirring. The mixture is stirred for about 5 hours at 80°C, and then demineralized water is added up to an end volume of about 14 litres. The obtained mixture is charged to an autoclave, and is left 10 days at 175°C under its autogenous pressure. The crystalline solid which is discharged is washed, dried and calcined at 550°C The X-rays and I.R. analyses show that the product obtained is titanium-silicalite.

To the so-prepared zeolite, the following are added: 370 g of tetra-ethyl-silicate with vigorous stirring, and 395 g of an aqueous solution of tetra-propyl-ammonium hydroxide at 12% by weight, and the resulting mixture is heated for 1 hour at 60°C; then, 1,620 g of demineralized water is added, and the resulting mixture is kept stirred for a further hour. A clear solution is thus obtained, in which 1000 g of the 4-zeolite obtained as described above, is carefully dispersed.

The milky suspension resulting from the dispersion is fed to a spray-drier (a NIRO ATOMIZER disk-atomizer; temperature of entering air = 300°C; temperature of exiting air = 120°C; chamber diameter = 1.5 metres), with compact microspheres being obtained, which have an average diameter close to 20 μm.

The atomized product is charged to a muffle under an $N_2$ blanket and is heated up to 550°C. After a two-hour stay at that temperature under nitrogen, the atmosphere is gradually changed from N$_2$ to air, and the product is maintained for a further two hours at 550°C under air. The obtained product has the following chemical molar composition:

0.02 TiO$_2$ : SiO$_2$

Example 2

To a jacketed reactor of 1 litre of capacity, equipped with a temperature-control system, mechanical stirring means and pressure control system, 350 g of methanol, 5.3 g of titanium-silicalite prepared as described in Example 1, and 39 g of butene-2 (cis-isomer 38%, trans-isomer 62%) are charged. To a supply vessel connected to the reactor, 15.3 g of 60%-H$_2$O$_2$ (C$_4$H$_8$/H$_2$O$_2$ = 2.6) is charged.

After regulating the temperature at the controlled value of 28°C, hydrogen peroxide is added to the suspension with strong stirring.

The disappearing of H$_2$O$_2$ is monitored by drawing at regular time intervals samples of the reaction mixture, which are submitted to the iodometric analysis.

After one hour, the residual hydrogen peroxide is less than 1%. The quantitative analysis of the reaction products (g.l.c. on a 1.8-metre long column packed with Parapak PS, with benzene as the internal standard) shows the following composition:
- cis-2,3-epoxy-butane :
0.230 mol
- trans-2,3-epoxy-butane :
0.034 mol
- hydrolysis products of the epoxy products :
0.0053 mol

After cooling down to -10°C, the reaction mixture is discharged and unreacted butene is distilled off and recovered (23.3 g; cis-2-butene = 6%, trans-2-butene = 94%).

The above said recovered butene is reacted with 250 g of methanol, 4.3 g of titanium-silicalite and 21.0 g of H$_2$O$_2$ at 60%, at the temperature of 32°C and with the above disclosed modalities. After 75 minutes, the conversion of H$_2$O$_2$ is of 96% and the reaction products have the following composition:
- cis-2,3-epoxy-butane :
0.023 mol
- trans-2,3-epoxy-butane :
0.331 mol
- solvolysis products :
0.0016 mol

Example 3

The following example is supplied for comparison purposes, but does not fall within the scope of the present invention.

To a jacketed reactor of 1 litre of capacity, equipped with a temperature-control system, mechanical stirring means and pressure control system, 350 g of methanol, 5.3 g of titanium-silicalite prepared as described in Example 1 and 39 g of butene-2 (cis-isomer 38%, trans-isomer 62%) are charged.

To a supply vessel connected to the reactor, 39 g of 60%-H$_2$O$_2$ (C$_4$H$_8$/H$_2$O$_2$ = 1.0) is charged.

After regulating the temperature at the controlled value of 28°C, hydrogen peroxide is added to the suspension with strong stirring.

The disappearing of H$_2$O$_2$ and the formation of the products is monitored as described in Example 2.

After 75 minutes, the conversion of H$_2$O$_2$ is of 95%, and the composition of the products is as follows:
- cis-2,3-epoxy-butane :
0.246 mol
- trans-2,3-epoxy-butane :
0.402 mol
- solvolysis products :
0.005 mol

Example 4

In the reactor of Example 2, and with the same modalities, 350 g of methanol, 5.5 g of titanium-silicalite prepared as in Example 1, 32 g of pentene-2 (35% cis-isomer, 65% trans-isomer) and 8.6 g of H$_2$O$_2$ at 60% (C$_5$H$_{10}$/H$_2$O$_2$ = 3.0) are reacted.

After 60 minutes at 25°C, the conversion of H$_2$O$_2$ is of 97%, with the composition of the reaction products being as follows:
- cis-2,3-epoxy-pentane :
0.140 mol
- trans-2,3-epoxy-pentane :
0.007 mol

Unreacted pentene is separated under reduced pressure from the reaction mixture (21.3 g) and is charged to a reactor similar to that of the first epoxidation, together with with 250 g of methanol, 4.5 g of titanium-silicalite and 16 g of H$_2$O$_2$ at 60%. After 55 minutes at the temperature of 25°C, the conversion of H$_2$O$_2$ is of 97%, with the reaction products having the following composition:
- cis-2,3-epoxy-pentane :
0.020 mol
- trans-2,3-epoxy-pentane :
0.249 mol

Example 5

The following example is supplied for comparison purposes, but does not fall within the scope of the invention.

In a similar way to as disclosed in Example 4, 350 g of methanol, 7.0 g of titanium-silicalite prepared as described in Example 1, 32 g of pentene-2 (cis-isomer 35%, trans-isomer 65%) and 24 g of $H_2O_2$ at 60% ($C_5H_{10}/H_2O_2$ = 1.0) are reacted.

After 75 minutes at 25°C, the conversion of $H_2O_2$ is of 96%, with the reaction products having the following composition:
- cis-2,3-epoxy-pentane :
0.142 mol
- trans-2,3-epoxy-pentane :
0.256 mol
- solvolysis products :
0.008 mol

Example 6

To a jacketed glass reactor equipped with a temperature-control system and mechanical stirring means, 100 g of methanol, 17 g of octene-2 (40% of cis-isomer, 60% of trans-isomer) and 1.5 g of titanium-silicalite prepared as described in Example 1 are charged. After regulating the temperature at the controlled value of 40°C, 3.4 g of 60%-$H_2O_2$ - ($C_8H_{16}/H_2O_2$ = 2.5 by mol) is added all together.

The reaction is monitored by drawing at regular time intervals samples of the reaction mixture, titrating hydrogen peroxide by the iodometric route, and analysing the reaction product by gas-chromatography.

After 50 minutes, the conversion of hydrogen peroxide is of 98%, with the reaction products having the following composition:
- cis-2,3-epoxy-octane :
0.0533 mol
- trans-2,3-epoxy-octane :
0.003 mol
- solvolysis products :
0.0025 mol

The suspension is cooled, the residual hydrogen peroxide is decomposed by means of the addition of Pd on silica, and the solid is finally filtered off.

Unreacted octene-2 is recovered by fractional distillation under reduced pressure (10.1 g) and is charged again to a reactor system analogous to that used for the first cycle, to react with 70 g of methanol, 1.2 g of titanium-silicalite and 4.9 g of $H_2O_2$ at 60%.

After 60 minutes at 40°C, the conversion of $H_2O_2$ is of 96% and the reaction products have the following composition:
- cis-2,3-epoxy-octane :
0.004 mol
- trans-2,3-epoxy-octane :
0.0760 mol

- solvolysis products :
0.003 mol

Example 7

The following example is supplied for comparison purposes, but does not fall within the scope of the invention.

In a similar way to as disclosed in Example 6, 17 g of octene-2 (cis-isomer 40%, trans-isomer 60%) and 8.6 g of $H_2O_2$ at 60% ($C_8H_{16}/H_2O_2$ = 10 by mol) are reacted in 100 g of methanol, and in the presence of 1.9 g of titanium-silicalite.

After 60 minutes at 40°C, the conversion is of 94%, with the reaction products having the following composition:
- cis-2,3-epoxy-octane :
0.054 mol
- trans-2,3-epoxy-octane :
0.084 mol
- solvolysis products :
0.004 mol

Example 8

To a jacketed glass reactor equipped with a temperature-control system, magnetic-driven stirring means, and pressure gauge, 40 g of ethanol at 95%, 4.0 g of butene-2 (42% of cis-isomer, 58% of trans-isomer) and 0.48 g of titanium-silicalite are charged. After regulating at the controlled temperature of 25°C, 2.6 cc of $H_2O_2$ at 33% (weight/volume) dissolved in 10 cc of ethanol is added with good stirring; $C_4H_8/H_2O_2$ = 2.8 by mol.

The reaction is monitored by submitting to analysis very small samples of the reaction mixture, drawn at regular time intervals.

Hydrogen peroxide is determined by means of the iodometric method, and the reaction products amounts are determined by gas-chromatography, with the internal-standard method.

After 40 minutes, the converted hydrogen peroxide is of 96%, with a selectivity to epoxides of 99% (relatively to $H_2O_2$). These latter are constituted by:
- cis-2,3-epoxy-butane :
0.0225 mol; and
- trans-2,3-epoxy- butane :
0.00144 mol

The amount of the hydrolysis product is negligible.

The reaction mixture is cooled down to -15°C, and the catalyst is filtered off.

Unreacted butene is recovered by distillation: 2.40 g (16% of cis-isomer, 84% of trans-isomer). It is subsequently charged to a reactor similar to the preceding one, together with 28 g of 95% ethanol, and 0.39 g of titanium-silicalite. After reaching the controlled temperature of 25°C, with stirring, 2.40 g of $H_2O_2$ at 60% is added.

After 50 minutes, the converted hydrogen peroxide is of 95%, with the epoxides being constituted by:
- trans-isomer :
0.035 mol and
- cis-isomer :
0.0052 mol

Example 9

This example is given for comparative purposes, but does not fall within the scope of the invention.

With the same modalities as disclosed in Example 8, the reaction of epoxidation is carried out at 25°C. To the reactor, 40 g of ethanol at 95%, 4.0 g of butene-2 (cis-isomer 42%, trans-isomer 58%) and 0.53 g of titanium silicalite are charged. After reaching the controlled temperature of 25°C, 4.0 g of $H_2O_2$ at 60% are added; $C_4H_8/H_2O_2$ = 1.011 by mol.

After 60 minutes, the converted hydrogen peroxide is of 95%, with a selectivity to epoxides of 98%. These latter are constituted by 0.029 mol of cis-2,3-epoxy-butane (44%) and 0.037 mol of trans-2,3-epoxy-butane (56%).

## Claims

1. Process for separately preparing the "cis" and the "trans epoxy derivatives of a mixture of "cis" and "trans" isomers of unsubstituted alkenes by epoxidizing such a mixture with hydrogen peroxide, or with a hydrogen-peroxide-evolving compound, in the presence of an epoxidation catalyst, characterized in that the epoxidation catalyst is a synthetic zeolite having the formula:

$$x.TiO_2.(1-x)SiO_2$$

wherein **x** is from 0,0001 to 0,04 , and in that the process is carried out stepwise, in the first step with an amount of hydrogen peroxide near to the molar amount of the "cis" isomer of the starting isomer mixture, whereby a predominantly "cis" epoxy derivative is obtained which, in the second step, is separated from the unreacted predominantly "trans" unsubstituted alkenes, these latter being subsequently independently subjected to epoxidation with hydrogen peroxide in a nearly equimolecular amount relative to said unreacted predominantly "trans" unsubstituted alkenes.

2. Process according to Claim 1, wherein the starting unsubstituted alkenes are selected from butene-2, pentene-2 and octene-2.

3. Process according to Claim 1, wherein the amount of hydrogen peroxide in the first reaction step is from 0,85 mol to 1,5 mol per mol of the starting "cis" isomeric unsubstituted alkenes.

4. Process according to Claim 1, wherein the amount of hydrogen peroxide in the second reaction step is from 0,85 mol to 1,5 mol per mol of the unreacted "trans" isomeric unsubstituted alkenes recovered from the first reaction step.

5. Process according to Claim 1, wherein the epoxidation temperature is from -20°C to 100°C.

6. Process according to Claim 5, wherein the epoxidation temperature is from 0°C to 70°C.

7. Process according to Claim 1, wherein the epoxidation reaction is carried out in a polar solvent medium selected from the alcohols, the glycols, the ketones, the ethers, the esters and water.

8. Process according to Claim 1, wherein the concentration of hydrogen peroxide, when used as as aqueous solution, is from 10% to 70% $H_2O_2$ on a weight-to-volume basis.

## Revendications

1. Procédé permettant de préparer séparément les dérivés époxy cis et trans d'un mélange d'isomères cis et trans d'alcènes non substitués, par époxydation d'un tel mélange avec du peroxyde d'hydrogène ou avec un composé dégageant du peroxyde d'hydrogène, en présence d'un catalyseur d'époxydation, caractérisé en ce que le catalyseur d'époxydation est une zéolithe synthétique de formule :

$$xTiO_2. (1-x) SiO_2$$

dans laquelle x vaut de 0,0001 à 0,04, et en ce que le procédé est effectué par étapes, avec une première étape dans laquelle on utilise une quantité de peroxyde d'hydrogène, exprimée en moles, voisine de la quantité d'isomères cis dans le mélange d'isomères de départ, ce qui permet d'obtenir un dérivé époxy principalement cis, qui, dans la seconde étape, est séparé d'avec les alcènes non substitués n'ayant pas réagi, principalement trans, ces derniers étant ensuite indépendamment soumis à une époxydation avec du peroxyde d'hydrogène utilisé en un nombre de moles à peu près égal à celui des alcènes non substitués n'ayant pas réagi, principalement trans.

2. Procédé conforme à la revendication 1, dans lequel les alcènes non substitués de départ sont choisis parmi le butène-2, le pentène-2 et l'octène-2.

3. Procédé conforme à la revendication 1, dans lequel la quantité de peroxyde d'hydrogène utilisée dans la première étape de réaction vaut de 0,85 mole à 1,5 mole par mole d'isomères cis des alcènes non substitués de départ.

4. Procédé conforme à la revendication 1, dans lequel la quantité de peroxyde d'hydrogène utilisée dans la seconde étape de réaction vaut de 0,85 mole à l,5 mole par mole d'isomères trans des alcènes non substitués n'ayant pas réagi, récupérés après la première étape de réaction.

5. Procédé conforme à la revendication 1, dans lequel la température d'époxydation vaut de -20°C à 100°C.

6. Procédé conforme à la revendication 5, dans lequel la température d'époxydation vaut de 0°C à 70°C.

7. Procédé conforme à la revendication 1, dans lequel la réaction d'époxydation est effectuée dans un solvant polaire choisi parmi les alcools, les glycols, les cétones, les éthers, les esters et l'eau.

8. Procédé conforme à la revendication 1, dans lequel, quand on utilise le peroxyde d'hydrogène sous forme de solution aqueuse, la concentration de celle-ci vaut de 10 % à 70 % d'$H_2O_2$, en poids par volume.

**Patentansprüche**

1. Verfahren zur getrennten Herstellung der cis- und der trans-Epoxyderivate eines Gemisches aus cis- und trans-Isomeren von unsubstituierten Alkenen durch Epoxidieren eines solchen Gemisches mit Wasserstoffperoxid oder mit einer Wasserstoffperoxid entwickelnden Verbindung in Gegenwart eines Epoxidationskatalysators, dadurch gekennzeichnet, daß der Epoxidationskatalysator ein synthetischer Zeolith mit der Formel:

$$x.TiO_2.(1-x)SiO_2$$

ist, worin x einen Wert von 0,0001 bis 0,04 aufweist, und daß das Verfahren stufenweise ausgeführt wird, in der ersten Stufe mit einer Wasserstoffperoxidmenge nahe der molaren Menge des cis-Isomers des eingesetzten Isomerengemisches, wodurch ein überwiegend cis-Epoxyderivat erhalten wird, das in der zweiten Stufe von den nicht umgesetzten, überwiegend trans-unsubstituierten Alkenen abgetrennt wird, welche letztgenannten anschließend gesondert einer Epoxidation mit Wasserstoffperoxid in nahezu äquimolekularer Menge, bezogen auf die nicht umgesetzten, überwiegend trans-unsubstituierten Alkene, unterworfen werden.

2. Verfahren nach Anspruch 1, worin die eingesetzten unsubstituierten Alkene unter Buten-2, Penten-2 und Octen-2 ausgewählt werden.

3. Verfahren nach Anspruch 1, worin die Wasserstoffperoxidmenge in der ersten Reaktionsstufe von 0,85 Mol bis 1,5 Mol je Mol eingesetzte unsubstituierte cis-Alkenisomere beträgt.

4. Verfahren nach Anspruch 1, worin die Wasserstoffperoxidmenge in der zweiten Reaktionsstufe von 0,85 bis 1,5 Mol je Mol nicht-umgesetzten, aus der ersten Reaktionsstufe gewonnenen unsubstituierten trans-Alkenisomeren beträgt.

5. Verfahren nach Anspruch 1, worin die Epoxidationstemperatur von -20°C bis 100°C beträgt.

6. Verfahren nach Anspruch 5, worin die Epoxidationstemperatur von 0°C bis 70°C beträgt.

7. Verfahren nach Anspruch 1, worin die Epoxidationsreaktion in einem polaren Lösungsmittelmedium, ausgewählt unter Alkoholen, Glykolen, Ketonen, Ethern, Estern und Wasser, ausgeführt wird.

8. Verfahren nach Anspruch 1, worin die Wasserstoffperoxidkonzentration bei Verwendung als wäßrige Lösung von 10 bis 70% $H_2O_2$, bezogen auf Gewicht/Volumen-Basis, beträgt.